# FASCICULE DE BREVET EUROPEEN

(11) **EP 0 451 565 B1**
(45) Date de publication et mention de la délivrance du brevet: **21.06.1995**
(21) Numéro de dépôt: 91104385.9
(22) Date de dépôt: 21.03.1991
(51) Int. Cl.: G07C 9/00, A61B 5/117

(54) **Procédé d'utilisation d'un ensemble de moyens aptes à rendre apparente une information complexe latente et installation pour la mise en oeuvre de ce procédé**
Verfahren zum Gebrauch einer Gesamtheit von Mitteln, die eine komplexe, latente Information sichtbar machen und Einrichtung zum Durchführen dieses Verfahrens
Method using an assembly of means capable of making visible complex latent information and installation for carrying out this method

(30) Priorité: 28.03.1990 CH 1026/90
(43) Date de publication de la demande: 16.10.1991
(73) Titulaire: Bauer, Eric, CH-2006 Neuchatel (CH)
(72) Inventeur: Bauer, Eric, CH-2006 Neuchatel (CH)
(74) Mandataire: Robert, Jean S.

(56) Documents cités:
- EP-A- 0 048 489
- WO-A-87/05790
- GB-A- 2 185 937

## Description

La présente invention a pour objet une installation comprenant un ensemble de têtes de lecture d'une information complexe latente, produisant la réflexion d'ultrasons envoyés sur le support de ladite information.

La lecture d'informations complexes latentes, notamment d'informations biométriques, pose des problèmes qui ont donné lieu à des solutions ne donnant pas toujours satisfaction.

Un des inconvénients des procédés connus réside dans le fait que tous les utilisateurs de telles installations, pour la lecture d'informations d'un même type, reçoivent la même "image", ce qui peut permettre des indiscrétions. En effet, une fois en possession de l'information rendue apparente, il est possible d'avoir accès à des stocks de données en relation avec l'information, qu'il pourrait y avoir avantage soit à conserver confidentiels, soit à ne mettre à disposition que partiellement, pour des personnes ou groupes de personnes déterminés. En d'autres termes, avec les procédés connus, il est possible de se livrer à des recoupements d'informations qui ne sont pas désirés. C'est ainsi, par exemple, qu'un stock de données relatives à un individu ne doit pas toujours être mis à la disposition, dans sa totalité, de toutes les entreprises, organisations ou collectivités auxquelles seules des parties de ce stock de données sont utiles.

Le but de la présente invention est de remédier à cet inconvénient en fournissant des moyens grâce auxquels les personnes habilitées à avoir accès à une partie d'un stock de données ne pourront pas avoir accès au reste des données de ce même stock.

Ce but est atteint grâce aux moyens définis dans la revendication 1.

Cependant, il est à remarquer que des moyens différents ont déjà été proposés dans un but du même genre. C'est ainsi que la demande de brevet PCT No WO 87/05790 décrit un dispositif de lecture d'information complexe, spécifiquement destiné à la lecture d'une information biométrique constituée par l'image du doigt d'un individu. De plus, l'information complexe préalablement lue dans une mémoire peut être codée avec des clés différentes afin de produire diverses images non identiques de l'information latente.

Le dessin représente, à titre d'exemple, plusieurs formes d'exécution de l'objet de l'invention.

La fig. 1 est une coupe schématique d'une tête de lecture connue, appartenant à l'état de la technique, permettant la lecture d'informations latentes.

La fig. 2 représente un mode d'utilisation de cette tête de lecture.

La fig. 3 est une coupe d'un détail de ladite tête de lecture, et d'une carte informatique avec laquelle elle est utilisée.

Les figs. 4 et 5 sont des coupes analogues à celle de la fig. 3 représentant des variantes de la carte informatique et de la tête de lecture correspondante.

La fig. 6 est une vue en perspective schématique d'une première forme d'exécution d'une tête de lecture d'installation de lecture d'informations latentes selon la présente invention.

La fig. 7 est une coupe d'une variante de tête de lecture, et

Les figs. 8 et 9 sont des vues en plan, de dessous, d'un détail de deux variantes de têtes de lecture.

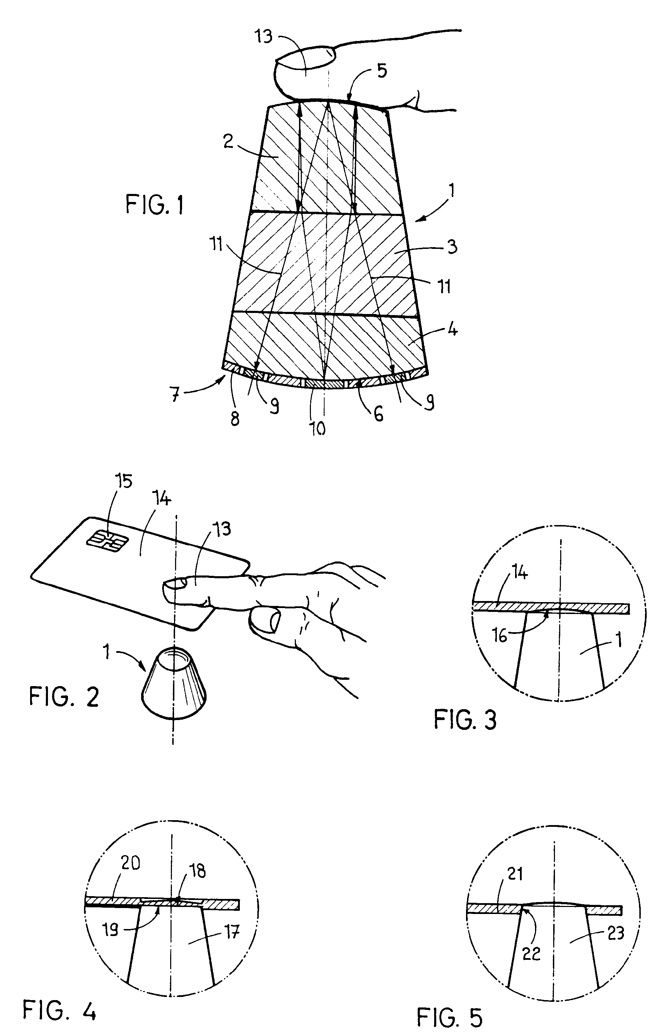

La tête de lecture représentée à la fig. 1, désignée d'une façon générale par 1, comprend un corps composite de forme générale tronconique formé de trois parties 2, 3 et 4, les deux premières en deux matières plastiques différentes et la troisième en un matériau actif, par exemple en piézo-céramique.

Les deux faces axiales de cette tête, désignées par 5 et 6, sont toutes deux convexes. La base 6 est revêtue d'une plaque de résonance, désignée d'une façon générale par 7, formée d'une masse diélectrique 8 dans laquelle sont noyés des pions métallisés 9 et 10 qui forment, avec la masse diélectrique, une capacité.

Une telle tête de lecture étant connue en soi, de même que son mode opératoire, elle ne sera pas décrite ici de façon détaillée. Qu'il suffise de dire que l'un des pions métallisés, en l'occurrence le pion central 10, est soumis à l'action d'un générateur d'ultrasons et que les ondes qu'il émet sont réfléchies par le doigt, désigné par 13, que la personne dont l'identité est à vérifier pose sur la face antérieure 5 de la tête, et renvoyées sur et par les différents pions 9 et 10 qui, par interaction, finissent par produire un signal image de l'information biométrique que contient le doigt 13.

Si une telle tête de lecture, coopérant avec une électronique adéquate, est utilisée par exemple pour observer le doigt d'un individu tel le doigt indiqué en 13 à la fig. 1, en contact avec la face convexe 5 de la tête de lecture, les ultrasons qui sont réfléchis par ce doigt fournissent des signaux que, par une électronique adéquate, l'on pourra transformer en une image affichée constituant une image biométrique différant d'un individu à l'autre.

C'est ainsi que l'on pourra observer le doigt du porteur d'une carte informatique telle que la carte 14 de la fig. 2 munie d'un microprocesseur 15 qui contient une information codée correspondant aux informations biométriques du porteur de cette carte. Tout abus dans l'emploi de la carte sera ainsi empêché dès lors qu'il faut, pour que la carte fonctionne, que l'information codée qu'elle contient corresponde à l'information latente que porte le doigt du porteur et que lit la tête de lecture.

Comme on le voit d'après la fig. 3, la carte informatique 14 présentera une zone circulaire concave 16 dans laquelle vient s'engager l'extrémité de la tête de lecture, et en regard de laquelle l'utilisateur de la carte posera le doigt.

Dans la variante de la fig. 4, la tête de lecture, désignée par 17, présente une extrémité, désignée par 18, qui est plane et qui vient se loger dans une partie en retrait 19 de la carte informatique, désignée par 20, en regard de laquelle l'utilisateur de la carte pose l'extrémité du doigt.

Quant à la variante de la fig. 5, elle comporte une carte informatique 21 percée d'une ouverture traversante 22 dans laquelle vient se loger l'extrémité de la tête de lecture, désignée par 23.

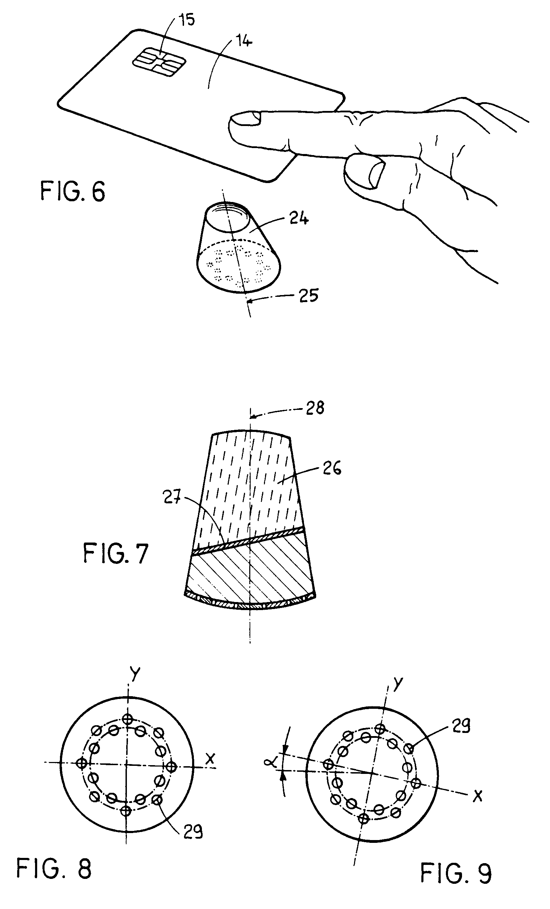

Selon les dispositions connues, la tête de lecture, tronconique, est symétrique par rapport à son axe longitudinal. Dans la première forme d'exécution de l'objet de l'invention, représentée à la fig. 6, la tête de lecture, désignée d'une façon générale par 24, est asymétrique par rapport à son axe longitudinal, désigné par 25. Il en résulte que l'information rendue apparente à l'aide de cette tête de lecture, à partir d'une information latente, est altérée ou distordue, l'image de l'information apparente n'étant pas la même que celle que l'on obtiendrait à l'aide d'une tête de lecture ordinaire, telle celle de la fig. 1, par exemple.

Ainsi, les différents utilisateurs d'un stock de données, individus, collectivités, entreprises, etc..., pourront être équipés d'installations de lecture munies de têtes de lecture différentes qui fourniront, à chaque catégorie d'utilisateur, une image propre de l'information initiale grâce à laquelle ils pourront n'avoir accès qu'à une partie des données stockées. De plus, ne disposant pas d'image non altérée, les utilisateurs ne pourront pas, par recoupement, avoir accès à l'ensemble des données stockées.

On pourra obtenir le même résultat en incluant, à l'intérieur de la tête de lecture, telle la tête 26 de la fig. 7, une plaque de diffraction 27 dont la présence, comme aussi l'inclinaison par rapport à l'axe longitudinal, désigné par 28, de la tête de lecture, fera varier l'image recueillie.

On pourra encore agir par le choix des matériaux constituant le corps de la tête de lecture et par le nombre de ces matériaux ou des plaques de diffraction d'une même tête, produisant ainsi des effets diffracteurs différents.

Dans tous les exemples susmentionnés, le but poursuivi --déformer l'information rendue apparente-- est obtenu par diffraction.

Cependant, on pourra aussi, dans le même but, user d'autres moyens.

C'est ainsi que l'on pourra modifier l'orientation angulaire, par rapport à l'axe longitudinal de la tête de lecture, de la plaque de résonance de celle-ci. Il faudra, pour que cela soit efficace, que les pions métallisés désignés par 29 aux figs. 8 et 9, ne soient pas disposés symétriquement par rapport au centre de la plaque de résonance. Dans ce cas, une modification angulaire de celle-ci, indiquée par l'angles α de la fig. 9, "gauchit" l'information fournie.

## Revendications

1. Installation comprenant un ensemble de têtes de lecture d'une information complexe latente, produisant la réflexion d'ultrasons envoyés sur le support de ladite information, caractérisée par le fait qu'au moins une partie de chacune desdites têtes de lecture (24; 26) est conformée de manière à produire une image altérée de l'information latente, les images altérées étant différentes d'une tête à l'autre.

2. Installation suivant la revendication 1, caractérisée par le fait que l'altération de l'image de l'information latente est produite par des modifications différentes d'une tête à l'autre dans la géométrie générale de la tête de lecture (24; 26).

3. Installation suivant la revendication 1, caractérisée par le fait que l'altération de l'image de l'information latente est produite par au moins une plaque de diffraction (27) que comporte chacune desdites têtes de lecture (26) et dont la position est différente, d'une tête à l'autre.

4. Installation suivant la revendication 3, caractérisée par le fait que les modifications de position de la plaque de diffraction (27) des têtes de lecture (26) résident dans une modification de l'inclinaison de ladite plaque de diffraction (27) par rapport à l'axe longitudinal de ladite tête (26).

5. Installation suivant la revendication 1, caractérisée par le fait que l'altération de l'image de l'information latente est produite par des modifications différentes d'une tête à l'autre du matériau dont sont faits les corps desdites têtes de lecture.

6. Installation suivant la revendication 5, caractérisée par le fait que les corps des têtes de lecture sont constitués de matériaux composites.

7. Installation suivant la revendication 1, dans laquelle les têtes de lecture comprennent des plaques de détection munies d'organes émetteurs et récepteurs, caractérisée par le fait que l'altération de l'image de l'information latente est produite par des modifications de position desdits organes émetteurs et récepteurs desdites plaques de détection.

## Patentansprüche

1. Einrichtung mit einer Gesamtheit von Leseköpfen zum Lesen einer komplexen, latenten Information, welche Ultraschall reflektiert, der auf den Träger der besagten Information gestrahlt wird, dadurch gekennzeichent, daß wenigstens ein Teil eines jeden Lesekopfes (24; 26) derart ausgebildet ist, daß er ein verzerrtes Bild der latenten Information gibt, wobei die verzerrten Bilder von einem Kopf zum anderen unterschiedlich sind.

2. Einrichtung nach Anspruch 1, dadurch gekennzeichnet, daß die Verzerrung des Bildes der latenten Information durch Veränderungen, welche von einem Kopf zum anderen unterschiedlich sind, der allgemeinen Geometrie des Lesekopfes (24; 26) bewirkt wird.

3. Einrichtung nach Anspruch 1, dadurch gekennzeichnet, daß die Verzerrung des Bildes der latenten Information mittels wenigstens einer Brechungsplatte (27) bewirkt wird, welche auf jedem Lesekopf (26) angebracht ist und deren Stellung, von einem Kopf zum anderen, unterschiedlich ist.

4. Einrichtung nach Anspruch 3, dadurch gekennzeichnet, daß die Veränderungen der Stellung der Brechungsplatte (27) der Leseköpfe (26) in einer Veränderung der Neigung der Brechungsplatte (27) gegenüber der Längsachse des Kopfes (26) bestehen.

5. Einrichtung nach Anspruch 1, dadurch gekennzeichnet, daß die Verzerrung des Bildes der latenten Information durch Veränderungen, welche von einem Kopf zum anderen unterschiedlich sind, des Materials, aus dem die Körper der Leseköpfe hergestellt sind, bewirkt wird.

6. Einrichtung nach Anspruch 5, dadurch gekennzeichnet, daß die Körper der Leseköpfe aus zusammengesetzten Materialien bestehen.

7. Einrichtung nach Anspruch 1, bei welcher die Leseköpfe Detektorplatten aufweisen, welche mit Sende- und Empfänger-Organen versehen sind, dadurch gekennzeichnet, daß die Verzerrung des Bildes der latenten Information durch Veränderungen der Stellung der Sende- und Empfangs-Organe der Detektorplatten bewirkt wird.

## Claims

1. Installation comprising a whole of reading heads of a latent complex information producing the reflection of ultrasounds sent on the support of the said information, characterized by the fact that at least a part of each of these reading heads (24; 26) is conformed in such a way as to produce an altered image of the latent information, the altered images being different from one head to another one.

2. Installation as claimed in claim 1, characterized by the fact that the alteration of the image of the latent information is produced by modifications different from one head to another one in the general geometry of the reading head (24; 26)

3. Installation as claimed in claim 1, characterized by the fact that the alteration of the image of the latent information is produced by at least a diffraction plate (27) with which each one of the said reading heads (26) is equipped, and the position of which is different from one head to another one.

4. Installation as claimed in claim 3, characterized by the fact that the variations of the position of the diffraction plate (27) of the reading heads (26) lie in a variation of the inclination of the said diffraction plate (27) with respect to the longitudinal axis of the head (26).

5. Installation as claimed in claim 1, characterized by the fact that the alteration of the image of the latent information is produced by variations different from one head to another one of the material of which are made the bodies of the said reading heads.

6. Installation as claimed in claim 5, characterized by the fact that the bodies of the reading heads are made of composite materials.

7. Installation as claimed in claim 1, in which the reading heads comprise plates of detection provided with emitting and receiving members, characterized by the fact that the alteration of the image of the latent information is produced by variations of the position of the said emitting and receiving members of the said plates of detection.
